(19) Europäisches Patentamt
European Patent Office
Office européen des brevets

(11) EP 1 498 112 A2

(12) DEMANDE DE BREVET EUROPEEN

(43) Date de publication:
**19.01.2005 Bulletin 2005/03**

(51) Int Cl.[7]: **A61K 7/135**

(21) Numéro de dépôt: **04291774.0**

(22) Date de dépôt: **12.07.2004**

(84) Etats contractants désignés:
**AT BE BG CH CY CZ DE DK EE ES FI FR GB GR HU IE IT LI LU MC NL PL PT RO SE SI SK TR**
Etats d'extension désignés:
**AL HR LT LV MK**

(30) Priorité: **16.07.2003 FR 0308672**

(71) Demandeur: **L'OREAL**
**75008 Paris (FR)**

(72) Inventeurs:
- **Rollat-Corvol, Isabelle**
  **75017 Paris (FR)**
- **Samain, Henri**
  **91570 Bievres (FR)**

(74) Mandataire: **Wattremez, Catherine**
**L'OREAL - D.I.P.I.**
**25-29 Quai Aulagnier**
**92600 Asnières (FR)**

# (54) Composition comprenant un polymere conducteur et un agent oxydant, procede de deformation permanente la mettant en oeuvre

(57) L'invention concerne une composition comprenant, dans un milieu cosmétiquement acceptable, (a) au moins un agent oxydant, (b) au moins un polymère conducteur de préférence comprenant au moins une unité répétitive de type des anilines, pyrroles, thiophènes ou bisthiophènes, furanes, para-phénylène-sulfures, para-phénylène vinylènes, indoles, amides aromatiques, hydrazides aromatiques, azométhines aromatiques, esters aromatiques particuliers.

Elle concerne de plus un procédé de traitement des fibres, notamment de décoloration, de déformation permanente ou de coloration, mettant en oeuvre une telle composition.

Elle a en outre pour objet l'utilisation d'une telle composition pour conférer un effet optique aux fibres kératiniques.

EP 1 498 112 A2

## Description

L'invention concerne une composition comprenant, dans un milieu cosmétiquement acceptable, au moins un polymère conducteur et au moins un agent oxydant. Elle concerne également un procédé de traitement de fibres kératiniques, plus particulièrement de décoloration, de déformation permanente ou de coloration, mettant en oeuvre la composition précitée. Elle a enfin pour objet l'utilisation d'une telle composition afin d'apporter un effet optique aux fibres kératiniques.

La présente invention a plus particulièrement trait au domaine des traitements de fibres kératiniques, de préférence humaines telles que notamment les cheveux. Plus précisément, l'invention concerne les compositions comprenant au moins un agent oxydant, et utilisées dans le cadre de traitements de fibres kératiniques.

Parmi ces traitements, on peut citer ceux mettant en jeu un composé oxydant, tels que les procédés de décoloration, de déformation permanente, ainsi que de coloration des fibres kératiniques.

En ce qui concerne la décoloration, ce traitement est effectué par oxydation du pigment "mélanine" aboutissant à la solubilisation et l'élimination partielle ou totale de ce pigment dans la fibre.

Dans ce but, on utilise des compositions décolorantes contenant un réactif peroxygéné, un agent alcalin comme activateur de ces sels peroxygénés. Ces compositions sont par la suite associées au moment de l'emploi à une composition aqueuse de peroxyde d'hydrogène.

Pour ce qui a trait aux procédés de déformation permanente, et plus spécialement à l'étape de fixation de tels procédés, on effectue cette dernière après la mise en forme des fibres (frisure ou lissage). Cette première étape, durant laquelle les ponts dissulfures présents dans les fibres sont ouverts, a lieu habituellement en présence d'un agent réducteur. Avant, après, ou simultanément à la réduction de ces ponts dissulfures, la fibre est mise en forme de la manière souhaitée (bigoudi, lissage). Une fois cette première étape effectuée, il est nécessaire de mettre en oeuvre une étape durant laquelle les ponts dissulfures sont reformés afin de stabiliser la forme obtenue.

Enfin, dans le domaine de la coloration, l'utilisation d'un agent oxydant est très souvent requise.

En effet, dans le domaine de la coloration d'oxydation, les composés utilisés sont des précurseurs de colorants d'oxydation, bases d'oxydation éventuellement associées à un ou plusieurs coupleurs. Ces composés sont des substances incolores ou peu colorées qui, par un processus de condensation oxydative, en présence d'un agent oxydant, conduisent à des composés qui colorent les fibres.

Dans le domaine de la coloration semi-permanente mettant en jeu des colorants directs, qui sont des composés colorants et colorés, la présence d'un agent oxydant est requise lorsque l'on souhaite obtenir une coloration éclaircissante.

Quel que soit l'objectif recherché (décoloration, déformation, coloration), ces procédés sont relativement agressifs envers la fibre kératinique et après ce genre de traitements, on peut observer à la longue une dégradation des fibres kératiniques, qui peut conduire à avoir des fibres plus ou moins rêches, cassantes, ternes.

Il est possible de traiter les fibres avec un agent apportant, par exemple, de la brillance aux fibres ; ledit agent pouvant soit être présent dans la composition de décoloration, dans la composition de fixation de permanente, ou encore dans la composition oxydante mise en oeuvre lors d'étapes de coloration, soit être apporté dans une composition appliquée après ces traitements.

Par exemple, pour donner de la brillance aux cheveux, on peut utiliser notamment des substances hydrophobes lubrifiantes, telle que des huiles ou des cires organiques ou des silicones. Toutefois, l'effet de brillance obtenu manque d'intensité et donne en général à la chevelure un aspect artificiel.

En outre, de telles compositions, une fois appliquées sur les cheveux, présentent l'inconvénient d'apporter un toucher gras ou collant à la chevelure.

Enfin, au cas où l'étape de décoloration ou de déformation permanente serait suivie d'une coloration, la présence de ces composés peut limiter la montée du colorant dans les fibres et par conséquent donner des colorations moins intenses, ou encore moins tenaces. Les mêmes inconvénients peuvent bien sûr être rencontrés lorsque la composition de coloration comprend ces composés.

La présente invention a donc pour but de proposer des compositions comprenant au moins un agent oxydant et au moins un polymère conducteur, qui apportent aux fibres kératiniques traitées, un effet optique particulier sans les inconvénients rencontrés avec les compositions classiques.

Par ailleurs, lorsque les polymères conducteurs absorbent dans le domaine du spectre visible, la composition selon l'invention permet de colorer les fibres sans qu'un traitement particulier de coloration, postérieur au traitement de mise en forme des fibres, soit nécessaire.

Dans le cas où la composition oxydante est employée dans un procédé de coloration, la présence de ces polymères peut permettre d'optimiser la teneur en précurseurs de colorants d'oxydation ou en colorants directs.

Enfin, les fibres kératiniques présentent de manière avantageuse un toucher doux et agréable.

La présente invention a ainsi pour premier objet une composition comprenant, dans un milieu cosmétiquement acceptable,

(a) au moins un agent oxydant,
(b) au moins un polymère conducteur.

L'invention concerne un procédé de traitement de fibres kératiniques dans lequel on applique la composition selon l'invention.

L'invention concerne de plus un procédé de décoloration des fibres kératiniques, notamment humaines, et plus particulièrement des cheveux, consistant à mettre en oeuvre les étapes suivantes :

a) on mélange avant l'application sur les fibres kératiniques, une composition selon l'invention exempte de peroxyde d'hydrogène avec une composition aqueuse comprenant du peroxyde d'hydrogène, en présence éventuellement d'un agent alcalin;
b) on applique sur les fibres kératiniques, sèches ou humides, le mélange obtenu à l'étape précédente, et on laisse pauser pendant une durée suffisante pour avoir l'effet de décoloration souhaité,
c) on rince éventuellement les fibres,
d) éventuellement on lave les fibres et on les rince,
e) on sèche ou on laisse sécher les fibres.

Selon une autre variante, le procédé selon l'invention est un procédé de déformation permanente des fibres kératiniques, notamment humaines, et plus particulièrement des cheveux, consistant à effectuer les étapes suivantes :

a) on applique sur des fibres kératiniques préalablement mises en forme avec une composition comprenant au moins un agent réducteur puis éventuellement rincées, une composition selon l'invention, pendant une durée suffisante pour avoir la fixation de la forme ;
b) on rince éventuellement les fibres,
c) éventuellement on lave les fibres et on les rince,
d) on sèche ou on laisse sécher les fibres.

L'invention concerne de plus un procédé de coloration des fibres kératiniques, notamment humaines, et plus particulièrement des cheveux, consistant à mettre en oeuvre les étapes suivantes :

a) on mélange avant l'application sur les fibres kératiniques, une composition tinctoriale comprenant au moins un précurseur de colorant d'oxydation et/ou au moins un colorant direct, avec une composition aqueuse selon l'invention ;
b) on applique sur les fibres kératiniques, sèches ou humides, le mélange obtenu à l'étape précédente, et on laisse pauser pendant une durée suffisante pour avoir l'effet de coloration souhaité,
c) on rince éventuellement les fibres,
d) éventuellement on lave les fibres et on les rince,
e) on sèche ou on laisse sécher les fibres.

L'invention a de même pour objet l'utilisation d'une composition comprenant au moins un agent oxydant et au moins un polymère conducteur pour conférer un effet optique aux fibres kératiniques.

En effet, la composition selon l'invention apporte à l'ensemble des fibres kératiniques, et de façon uniforme, un effet optique particulier, et notamment une brillance substantiellement plus intense, plus naturelle, et plus esthétique qu'avec les moyens de l'art antérieur.

Par ailleurs, lorsque les polymères conducteurs présents dans la composition selon l'invention absorbent dans le spectre visible, on obtient simultanément un effet optique, comme de la brillance, et de la couleur.

Mais d'autres caractéristiques et avantages de l'invention apparaîtront plus clairement à la lecture de la description et des exemples qui suivent.

Dans ce qui va suivre et à moins d'une indication différente, les bornes d'un domaine de valeurs sont comprises comme faisant partie de ce domaine.

Au sens de la présente invention, le terme effet optique recouvre des effets de brillance, de couleur, métallique, goniochromatique, moiré.

Par ailleurs, et plus particulièrement, il est à noter que la brillance correspond à l'intensité lumineuse réfléchie sous un angle $\alpha$ lorsque la mèche de cheveux est éclairée sous un angle $-\alpha$. L'angle $\alpha$ classiquement utilisé pour mesurer cette réflexion spéculaire, autrement dit la brillance, est égal à 20°. Cet apport de brillance peut être mesuré par utilisation d'un brillancemètre comme il est par exemple décrit dans la norme ISO 2813 - 1994 de l'AFNOR (août 1994, rectificatif février 1997).

Polymères conducteurs

Selon la présente invention, on entend par "polymère conducteur" une structure moléculaire dans laquelle le ou les monomères présentent une forte délocalisation électronique et dont la disposition dans le squelette du polymère permet aux orbitales $\pi$ de se recouvrir. Cette caractéristique chimique se traduit par un phénomène de conduction électrique qui s'accompagne ou non d'un phénomène d'absorption dans le spectre UV-visible, voire dans l'infrarouge.
Par polymère conducteur absorbant dans le visible, on entend au sens de la présente invention, tout polymère conducteur présentant une absorbance non nulle dans le domaine de longueur d'ondes allant de 400 à 800 nm, même si les maxima d'absorption du polymère se situent en dehors de cette gamme.
Les polymères conducteurs mis en oeuvre dans le cadre de la présente invention sont des polymères conducteurs solubles ou dispersibles dans le milieu cosmétique approprié à l'application.
On dit que le polymère est soluble dans le milieu lorsqu'il forme un liquide limpide isotrope à 25°C dans le milieu comprenant de l'eau ou un mélange eau/solvant ; ceci étant obtenu dans tout ou partie d'une gamme de concentration comprise entre 0,01 et 50 % en poids de polymère conducteur.
De façon préférée, les polymères conducteurs mis en oeuvre dans le cadre de la présente invention sont des polymères conducteurs solubles ou dispersibles dans un milieu aqueux, avantageusement dans l'eau.
On dit que le polymère est dispersible dans le milieu comprenant de l'eau ou un mélange eau/solvant si, à 0,01% en poids, à 25°C, il forme une suspension stable de fines particules, généralement sphériques. La taille moyenne des particules constituant ladite dispersion est inférieure à 1 µm et, plus généralement, varie entre 5 et 400 nm, de préférence de 10 à 250 nm. Ces tailles de particules sont mesurées par diffusion de lumière.
Il est à noter que de manière avantageuse, ces polymères ne nécessitent pas l'emploi d'un agent dispersant.
De préférence, les polymères conducteurs se présentent sous une forme soluble dans le milieu de la composition.
De plus, les polymères présentent de manière avantageuse, une conductivité comprise entre 10-5 et 5.105 siemens/cm, plus particulièrement comprise entre 10-3 et $10^5$ siemens/cm, et de préférence comprise entre $10^{-1}$ et $10^4$ siemens/cm.
La conductivité est mesurée à l'aide d'un générateur de courant (RM2 Test Unit commercialisé par la société Jandel) muni d'une tête de mesure dite quatre pointes (Universal four-point probes commercialisé par la société Jandel). Les quatre pointes alignées et distantes du même espacement d sont appliquées par simple pression sur l'échantillon à analyser. Un courant I est injecté par les pointes externes à l'aide de la source de courant créant ainsi une variation de potentiel. La tension U est mesurée entre les deux pointes internes reliées au voltmètre du générateur de courant.
Dans cette configuration la conductivité de l'échantillon exprimée en S/cm est donnée par l'expression suivante :

$$\sigma = (K \times I) / (U \times e)$$

Avec :

K coefficient dépendant de la position des contacts sur la surface de l'échantillon.
Lorsque les pointes sont alignées et équidistantes, K est égal à : $\Pi/\log(2)$
I : valeur du courant injecté exprimé en ampères
U : valeur de la tension mesurée exprimée en volts
e : épaisseur de l'échantillon exprimée en cm.

Cette expression ne peut être utilisée que lorsque l'épaisseur du matériau est négligeable devant la distance d existant entre deux pointes (e/d < 0,25). Pour obtenir des épaisseurs suffisamment faibles et ainsi pouvoir calculer la conductivité du matériau, il est préconisé de réaliser la mesure sur un support non conducteur (par exemple, une lame de verre) recouvert du matériau à analyser obtenu par évaporation d'une solution diluée. Afin d'améliorer l'homogénéité du revêtement à analyser, il est également préconisé d'utiliser la technique de dépôt dite du spin coating.
Selon un mode de réalisation particulier, les polymères conducteurs présents dans la composition sont choisis parmi les polymères comprenant au moins une unité répétitive de formules suivantes :

les anilines de structure (I) suivante :

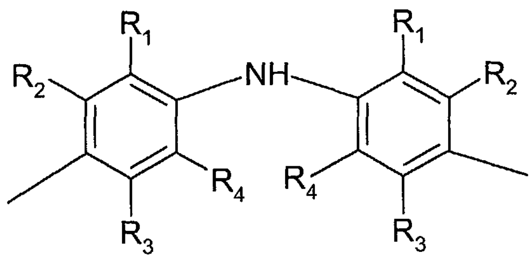

(I)

les pyrroles de structure (IIa) et (IIb) suivantes :

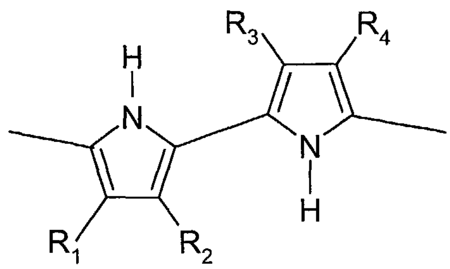

(IIa)

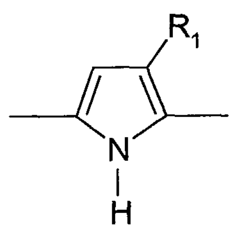

(IIb)

les thiophènes ou bisthiophènes de formules (IIIa), (IIIb) et (IIIc) suivantes :

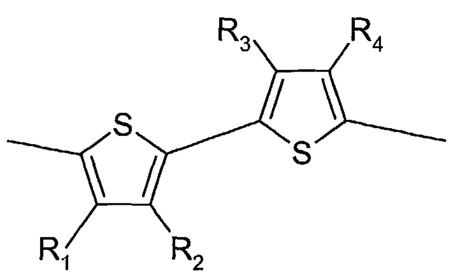

(IIIa)

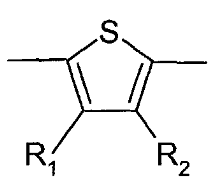

(IIIb)

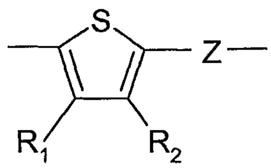

(IIIc)

les furanes de formule (IV) suivante :

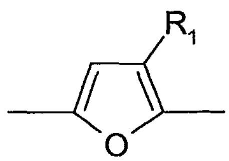

(IV)

les para-phénylène-sulfure de structure (V) suivante :

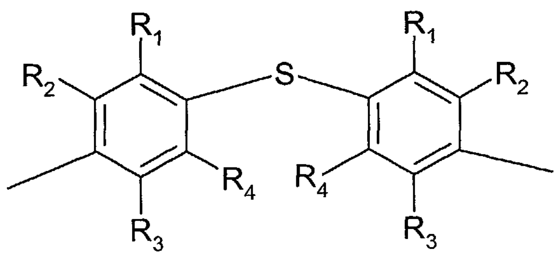

(V)

les paraphénylène vinylène de formule (VI) suivante :

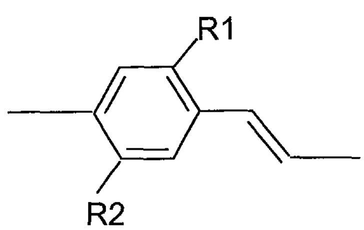

(VI)

les indoles de formule (VII) suivante :

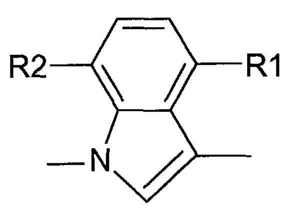

(VII)

les amides aromatiques de formules suivantes (VIIIa), (VIIIb), (VIIIc), (VIIId) :

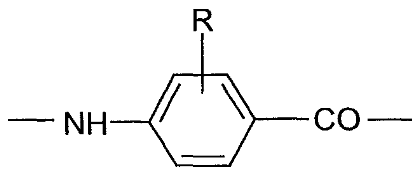

(VIIIa)

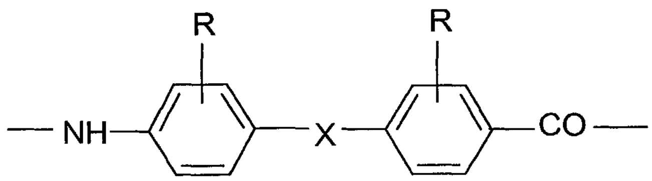

(VIIIb)

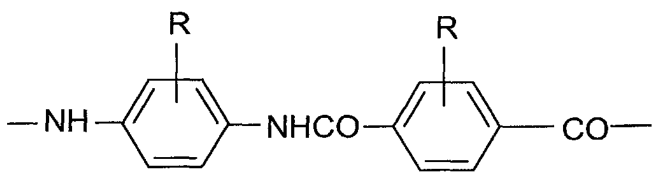

(VIIIc)

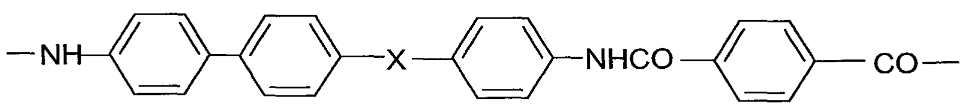

(VIIId)

les hydrazides aromatiques de formules (IXa), (IXb) et (IXc) suivantes :

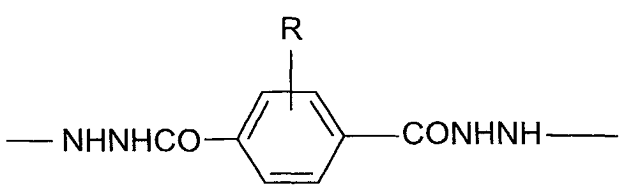

(IXa)

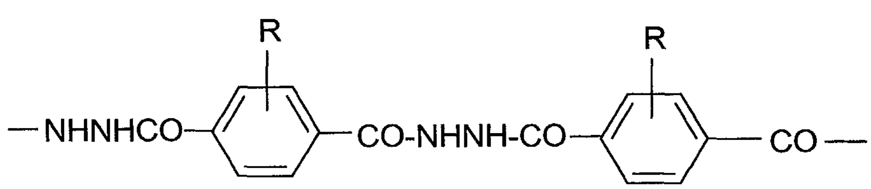

(IXb)

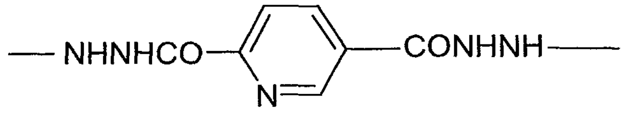

(IXc)

les azométhines aromatiques de formules (Xa), (Xb) et (Xc) suivantes :

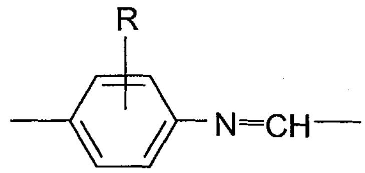

(Xa)

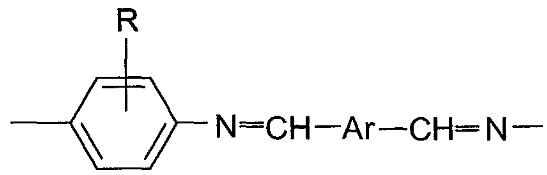

(Xb)

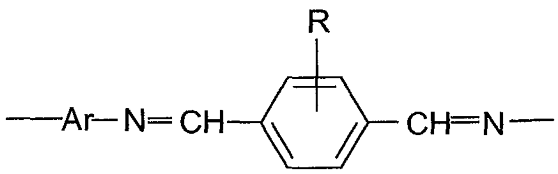

(Xc)

<u>les esters aromatiques de formules (XIa), (XIb) et (XIc) suivantes :</u>

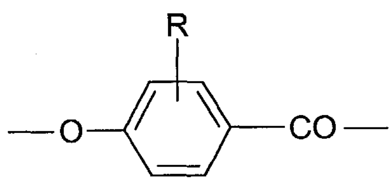

(XIa)

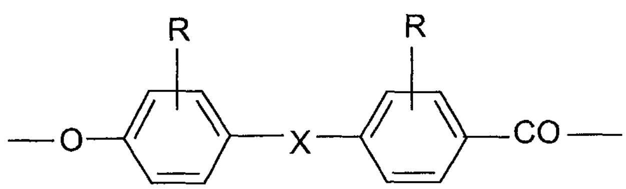

(XIb)

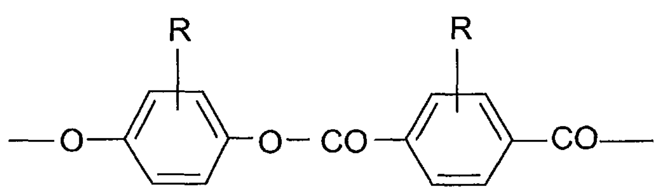

(XIc)

formules (I) à (XI) dans lesquelles :

les radicaux R, $R_1$ à $R_4$ identiques ou différents, sont choisis dans le groupe formé par l'hydrogène, un radical -R', -OR', -COOR', -OCOR', avec R' représentant un radical alkyle linéaire ou ramifié en $C_1$-$C_{20}$, un atome d'halogène, un radical nitro, un radical cyano, un radical cyanoalkyle, et un groupement solubilisant ;
Ar représente un radical comprenant un radical monoaromatique ou polyaromatique X = -NHCO-, -O-, -S-, -$SO_2$-, -N=N-, -$C(CH_3)_2$-, -$CH_2$-, -CH=CH-, -CH=N-;
Z= —CH=CH— ou —C≡C—.

Plus particulièrement. Ar représente au moins un radical choisi parmi les suivants :

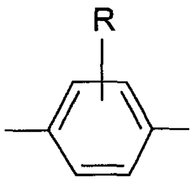 , 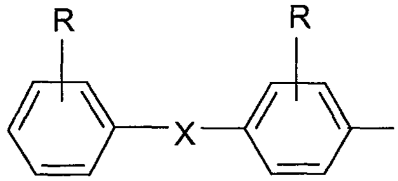 , 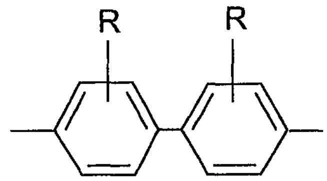 .

Par groupement solubilisant, on entend au sens de la présente invention, un groupement qui assure la solubilisation de ladite molécule dans le milieu cosmétique, de façon que le polymère présente un caractère conducteur après séchage de la composition.

Il est clair que le polymère conducteur présent dans la composition selon l'invention peut comprendre une ou plusieurs unités répétitives comprenant un ou plusieurs groupements solubilisants, et d'une ou plusieurs autres qui en sont dépourvues.

Les groupements solubilisants sont de préférence choisis dans le groupe formé par :

- un radical carboxylique (-COOH), carboxylate (-COO-$M^+$ avec M représentant un métal alcalin comme le sodium, le potassium, un métal alcalino-terreux, une amine organique telle que une amine primaire, secondaire ou tertiaire, une alcanolamine, un acide aminé),
- un radical sulfonique (-$SO_3H$), sulfonate (-$SO_3^-$ $M^+$, M ayant la même définition que ci-dessus),
- un radical amine primaire, secondaire, tertiaire,
- un radical ammonium quaternaire tel -$NR'_3{}^+$ $Z^-$ avec Z= Br, Cl, alkyl($C_1$-$C_4$)-$OSO_3$ et R' alkyles identiques ou non, linéaires ou ramifiés en $C_1$ à $C_{20}$, ou formant un hétérocycle avec l'azote pour deux d'entre eux,
- un radical hydroxyle,
- un radical polyoxyde d'alkylène en $C_2$-$C_3$.

Les fonctions acides carboxyliques ou sulfoniques peuvent ou non être neutralisées par une base, telle que l'hydroxyde de sodium, l'amino-2 méthyl-2 propanol, la triéthylamine ou encore la tributylamine, par exemple.
Les radicaux amines peuvent ou non être neutralisés par un acide minéral, tel que l'acide chlorhydrique, ou par un acide organique, tel que les acides acétique ou lactique, par exemple.

En outre, il est à noter que lesdits radicaux solubilisants peuvent être reliés au cycle par l'intermédiaire d'un groupement espaceur tel que par exemple un radical -R"-, -OR"-, - OCOR"- ou encore -COOR"- avec R" représentant un radical alkyle, linéaire ou ramifié en $C_1$-$C_{20}$, comprenant éventuellement un ou plusieurs hétéroatomes, tels que l'oxygène par exemple.

De préférence les radicaux R, $R_1$ à $R_4$ identiques ou différents, sont choisis parmi l'hydrogène, R', -OR', -OCOR', -COOR' avec R' représentant un radical alkyle linéaire ou ramifié en $C_1$-$C_6$, et parmi les groupements solubilisants suivants, neutralisés ou non : - COOH, -$CH_2COOH$, -$CH_2OH$, -$(CH_2)_6OH$, -$(CH_2)_3SO_3H$, -$O(CH_2)_3SO_3H$, - O $(CH_2)_3N(CH_2CH_3)_2$, -$[(CH_2)_2O]xCH_2CH_2OH$, -$[(CH_2)_2O]_xCH_2CH_2OCH_3$ avec x nombre moyen compris entre 0 et 200.

Le nombre d'unités répétitives du polymère n varie habituellement de 5 à 10000, notamment de 5 à 1000, plus particulièrement de 10 à 1000 et de préférence de 20 à 700.

Plus particulièrement, le polymère conducteur est tel qu'au moins un radical parmi R, R1 à R4 désigne un groupement solubilisant.

Conformément à un mode de réalisation particulier de l'invention, le polymère conducteur mis en oeuvre comporte au moins un groupement solubilisant par unité répétitive. Ainsi, de préférence, au moins un radical parmi R, $R_1$ à $R_4$ désigne un groupement solubilisant.

De préférence, le polymère conducteur est soluble dans le milieu de la composition.

Les polymères conducteurs présents dans la composition selon l'invention sont bien connus de l'homme de l'art et décrits notamment dans l'ouvrage "Handbook of organic conductive molecules and polymers" - Wiley 1997-New York, Vol 1, 2, 3, mais aussi dans la revue Can. J. Chem. Vol 64, 1986.

Les polythiophènes et leur synthèse sont plus particulièrement décrits dans l'article tiré de la revue Chem. Mater. 1998, Vol.10, N°7 pages 1990-1999 - par les auteurs RASMUSSEN S.C., PICKENS J.C. et HUTCHISON J.E. "A new, general approach to tuning the properties of functionalized polythiophenes : The oxidative polymerization of monosubstituted bithiophenes" ; dans l'article tiré de la revue Macromolecules 1998, 31, pages 933-936, par les mêmes auteurs "Highly conjugated, water-soluble polymers via direct oxidative polymerization of monosubstituted bithiophenes". Outre la polymérisation par oxydation chimique ou électrochimique, ils peuvent être aussi obtenus par polycondensation (thiophène dihalogéné ; catalyse avec des complexes de nickel ou de palladium) ; par couplage de Suzuki (couplage entre une fonction halogène, brome par exemple et un acide boronique, catalyse : complexe de palladium et base ; on a alors couplage de type AA-BB (réaction de monomères de type A-X-A avec B-X'-B) ou de type A-B (réaction de plusieurs monomères de type A-X-B) ; par couplage de Stille (formation de liaison carbone-carbone en présence d'une catalyse à base de Pd- type AA-BB ou A-B) ; par polymérisation de Reike (organozinc en présence d'un complexe de nickel) ; par polymérisation de type McCulloch, etc.

Les polymères conducteurs présents dans la composition selon l'invention sont par ailleurs décrits dans la demande internationale WO 99/47570.

Parmi les polymères conducteurs convenables à la réalisation de la présente invention, on peut citer plus particulièrement les polymères correspondant aux formules (IIIa), (IIIb) et (IIIc) dans lesquelles les groupes solubilisants sont de préférence un groupement acide carboxylique ; un groupement acide sulfonique ; un radical amine tertiaire ; un radical ammonium quaternaire tel que $-NR'_3{}^+ Z^-$ avec Z= Br, Cl, alkyl($C_1$-$C_4$)-$OSO_3$ et R' alkyles identiques ou non, linéaires ou ramifiés en $C_1$ à $C_{20}$, ou formant un hétérocycle avec l'azote pour deux d'entre eux ; lesdits groupes étant éventuellement reliés au cycle par un espaceur. Les fonctions acides carboxyliques ou sulfoniques peuvent ou non être neutralisées.

Ainsi, la polymérisation peut être réalisée par oxydation chimique ou électrochimique du monomère thiophène correspondant ou bien encore par polycondensation.

A titre d'illustration, les polythiophènes de formules (IIIa) et (IIIb) peuvent être obtenus par polymérisation par oxydation (par exemple avec une catalyse $FeCl_3$); par polycondensation de thiophène dihalogéné catalysée par des complexes de nickel ou de palladium (ex. : $NiCl_2(dppe)_2$); par couplage de Suzuki (couplage entre une fonction halogène, brome par exemple et un acide boronique, catalyse : complexe de palladium et base ; on a alors couplage de type AA-BB (réaction de monomères de type A-X-A avec B-X'-B ) ou de type A-B (réaction de plusieurs monomères de type A-X-B)); par couplage de Stille (formation de liaison carbone-carbone en présence d'une catalyse à base de Pd- type AA-BB ou A-B) ; par polymérisation de Reike (organozinc en présence d'un complexe de nickel) ; par polymérisation de type McCulloch, etc.

Les polythiophènes vinylènes de formule (IIIc) avec Z représentant —CH=CH—, peuvent notamment être obtenus par polymérisation de Gilch en présence d'une base forte (tertiobutylate de potassium), de 2,5 bis (bromoalkylène) thiophène ; par polymérisation par la méthode de Wessling via l'utilisation de précurseur à base de sels de sulfonium et pyrolyse ; par réaction de Wittig Wittig-Horner.

Les polythiophènes éthynylènes de formule (IIIc) avec Z représentant —C≡C— peuvent être obtenus par couplage de Heck-Sonogashira (de type AA-BB ou A-B ; formation de liaison carbone-carbone entre une fonction acétylénique terminale (ou acétylénique vraie) et une fonction bromo ou iodo catalysée par un complexe de palladium/cuivre ($PdCl_2(PPh_3)_3$, CuI ou $Cu(Oac)_2$) en présence d'une base telle que la triéthylamine, diisopropylamine, piperidine etc...) ; par méthathèse d'alkynes en présence d'un complexe de molybdène (de Mo(CO)6).

En général la fonctionnalisation des polythiophènes, en d'autres termes, l'apport du ou des groupes solubilisants ou non, est réalisée sur le monomère avant sa polymérisation.

Dans certains cas, l'obtention du groupe solubilisant est obtenu par suite de traitement de polymère. C'est notamment le cas de la fonction acide carboxylique qui peut être obtenue par hydrolyse de l'ester correspondant.

De préférence, les groupes solubilisants sont choisis parmi les groupements acide carboxylique ; acide sulfonique ; les radicaux amine tertiaire ; ammonium quaternaire tels que $-NR'_3{}^+ Z^-$ avec Z= Br, Cl, alkyl($C_1$-$C_4$)-$OSO_3$ et R', identiques ou non, représentant un radical alkyle linéaire ou ramifié en $C_1$-$C_{20}$ ; éventuellement relié au cycle par un espaceur, de préférence un radical alkyle en $C_1$-$C_{20}$ ; ainsi que leurs sels.

Les fonctions acides carboxyliques ou sulfoniques peuvent ou non être neutralisées.

Selon un mode de réalisation particulier de l'invention, le polymère conducteur correspond aux formules (IIIa), (IIIb) ou (IIIc), dans lesquelles au moins un radical R1 à R4 de la formule (IIIa) ou R1 ou R2 des formules (IIIb) ou (IIIc) représente un groupement solubilisant du type acide carboxylique, sous forme neutralisée ou non, éventuellement relié au cycle par un espaceur, de préférence un radical alkyle linéaire ou ramifié en C1-C20, le ou les autres radicaux représentant un atome d'hydrogène.

Les polymères conducteurs sont généralement présents dans la composition dans des proportions d'au moins 0,001 % en poids, plus particulièrement d'au moins 0,01 % en poids, de préférence d'au moins 0,1 % en poids et de manière encore plus préférée, d'au moins 0,5 % en poids, par rapport au poids total de la composition. Par ailleurs, la teneur en polymère conducteur est avantageusement d'au plus 50 % en poids, plus particulièrement d'au plus 30 % en poids, de préférence d'au plus 20 % en poids et de manière encore plus préférée d'au plus 10 % en poids, par rapport au poids total de la composition.

Selon un mode de réalisation particulièrement avantageux de l'invention, la teneur en polymère conducteur est comprise entre 0,1 et 50% en poids, plus particulièrement entre 0,1 et 30% en poids, et de préférence entre 0,5 et 10 % en poids, par rapport au poids total de la composition.

Comme indiqué auparavant, la composition selon l'invention comprend, outre ledit polymère conducteur, au

moins un agent oxydant.

Agent oxydant

Selon une première variante de l'invention, l'agent oxydant présent dans la composition, est choisi parmi les composés peroxygénés tels que les persulfates, perborates et percarbonates, d'ammonium ou de métaux alcalins, le peroxyde d'urée ; parmi les dérivés halogénés comme l'iode, les bromates de métaux alcalins, les hypochlorites de métaux alcalins par exemple ; parmi les ferricyanures de métaux alcalins ; parmi les enzymes telles que les peroxydases et les oxydo-réductases à deux ou à quatre électrons, seuls ou en mélanges.
Selon une deuxième variante, la composition comprend du peroxyde d'hydrogène en tant qu'agent oxydant.
Une troisième variante correspond à la combinaison des deux précédentes.
Plus particulièrement, la teneur totale en agent oxydant conforme à la première et à la deuxième variante, représente de 0,05% à 25% en poids par rapport au poids total de la composition.
Selon un mode de réalisation particulier de l'invention, la composition peut comprendre au moins un agent alcalin, dans les cas où la composition selon l'invention ne comprend pas de peroxyde d'hydrogène, ou ne comprend pas à la fois de l'eau et un sel peroxygéné.
Habituellement, ledit agent alcalin est choisi parmi l'urée, les silicates et phosphates de métaux alcalins ou alcalino-terreux, les composés précurseurs d'ammoniac, les carbonates alcalins, les alcanolamines telles que les mono-, di- et triéthanolamines ainsi que leurs dérivés, les hydroxydes de sodium ou de potassium et les composés de formule (A) suivante :

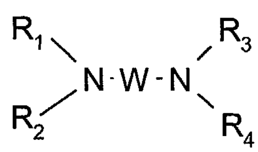
(A)

dans laquelle W est un reste propylène éventuellement substitué par un groupement hydroxyle ou un radical alkyle en $C_1$-$C_6$ ; $R_1$, $R_2$, $R_3$ et $R_4$, identiques ou différents, représentent un atome d'hydrogène, un radical alkyle en $C_1$-$C_6$ ou hydroxyalkyle en $C_1$-$C_6$.
La teneur en agent alcalin est habituellement comprise entre 0,01 et 40% en poids, de préférence entre 0,1 et 30% en poids, par rapport au poids total de la composition.
La composition peut aussi comprendre des agents tensioactifs non ioniques, anioniques, cationiques, amphotères ou zwitterioniques et parmi ceux-ci, on peut citer les alkylsulfates, les alkylbenzènesulfates, les alkyléthersulfates, les alkylsulfonates, les sels d'ammonium quaternaires, les alkylbétaïnes, les alkylphénols oxyéthylénés, les alcanolamides d'acides gras, les esters d'acides gras oxyéthylénés, ainsi que d'autres tensioactifs non-ioniques du type hydroxypropyléther.
Lorsque la composition comprend un ou plusieurs tensioactifs, leur teneur est habituellement de moins de 30% en poids, et de préférence comprise entre 0,5 et 10% en poids, par rapport au poids de la composition.
Le pH de la composition est généralement compris entre 1,5 et 12 environ, et de préférence entre 2,5 et 11 environ.
Il peut être ajusté à la valeur désirée au moyen d'agents acidifiants ou alcalins.
Les agents alcalins ont été décrits plus haut.
Quant aux agents acidifiants, on peut citer, à titre d'exemples, les acides minéraux ou organiques comme l'acide chlorhydrique, l'acide orthophosphorique, l'acide sulfurique ; les acides organiques tels que les acides sulfoniques, les acides carboxyliques comme l'acide acétique, l'acide tartrique, l'acide citrique, l'acide lactique.
La composition peut également comprendre divers adjuvants utilisés classiquement, tels que des agents épaississants ou gélifiants comme la silice pyrogénée à caractère hydrophile ou hydrophobe, les polymères associatifs non ioniques; anioniques ou amphotères, les polymères épaississant hydrosolubles d'origine synthétique ou naturelle comme la polyvinylpyrrolidone, l'acide polyacrylique, le polyacrylamide, les polysaccharides d'origine animale végétale ou microbienne.
Avantageusement, la teneur en agents épaississant est comprise entre 0,01 et 10% en poids, de préférence entre 0,05 et 5 % en poids, par rapport au poids total de la composition.
La composition peut de même comprendre divers adjuvants classiquement utilisés tels que des agents de conditionnement notamment cationiques ou amphotères de préférence anhydres ; des charges comme l'argile, la silice amorphe ; des liants comme la vinylpyrrolidone ; des lubrifiants comme les stéarates de polyol ou les stéarates de métaux alcalins ou alcalino-terreux ; des agents de contrôle du dégagement d'oxygène tels que le carbonate ou l'oxyde

de magnésium ; des agents colorants ; des agents matifiants comme les oxydes de titane ; des agents antioxydants ; des agents de pénétration; des agents séquestrants ; des parfums ; des tampons, des agents dispersants ; des agents filmogènes, des céramides ; des agents conservateurs ; des agents stabilisants, etc.

Généralement, la teneur en ces adjuvants ne dépasse pas 60 % en poids par rapport au poids de la composition.

La composition selon l'invention peut se présenter sous diverses formes.

Une première variante est constituée par une composition sous la forme d'une composition dépourvue d'eau ou comprenant une teneur en eau faible. Plus précisément, on considère comme faible une teneur en eau inférieure à 1 % en poids, de préférence inférieure à 0,5 % en poids par rapport au poids de la composition oxydante.

Dans le cadre de cette variante, la composition se présente plus particulièrement sous la forme d'une pâte, de granulés.

Une deuxième variante est constituée par des compositions comprenant un milieu cosmétiquement acceptable qui correspond plus particulièrement à de l'eau ou à un mélange d'eau et d'un ou plusieurs solvants organiques acceptables dans le domaine.

Parmi les solvants utilisables, on peut citer plus particulièrement, les alcools en C1-C4, tels que l'alcool éthylique, l'alcool isopropylique, les alcools aromatiques comme l'alcool benzylique, et l'alcool phényléthylique, ou les glycols ou éthers de glycol tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, le dipropylèneglycol ainsi que les alkyléthers de diéthylèneglycol comme par exemple, le monoéthyléther ou le monobutyléther du diéthylèneglycol, ou encore les polyols comme le glycérol. On peut également utiliser comme solvant les polyéthylèneglycols et les polypropylèneglycols, et les mélanges de tous ces composés.

Ces solvants, s'ils sont présents, représentent habituellement de 1 à 40 % en poids, plus préférentiellement de 5 à 30 % en poids, par rapport au poids total de la composition.

La présente invention a de plus pour objet une composition prête à l'emploi comprenant la composition selon l'invention comprenant au moins un composé peroxygéné et/ou des bromates de métaux alcalins, et du peroxyde d'hydrogène, en tant qu'agents oxydants.

Les procédés selon l'invention vont maintenant être décrits. Ils sont applicables à des fibres kératiniques, notamment humaines, telles que par exemple les cheveux.

Ainsi le procédé selon l'invention consiste à appliquer sur les fibres kératiniques, la composition qui vient d'être décrite.

Conformément à une première possibilité, le procédé est un procédé de décoloration consistant à effectuer les étapes suivantes :

a) on mélange avant l'application sur les fibres kératiniques, une composition selon l'invention ne comprenant pas de peroxyde d'hydrogène, avec une composition aqueuse comprenant du peroxyde d'hydrogène, éventuellement en présence d'un agent alcalin ;
b) on applique sur les fibres kératiniques, sèches ou humides, le mélange obtenu à l'étape précédente, et on laisse pauser pendant une durée suffisante pour avoir l'effet de décoloration souhaité,
c) on rince éventuellement les fibres,
d) éventuellement on lave les fibres et on les rince,
e) on sèche ou on laisse sécher les fibres.

Lors de l'étape a) la composition selon l'invention est mélangée avant l'application sur les fibres, avec une composition aqueuse de peroxyde d'hydrogène.
De préférence, la composition selon l'invention comprend, à titre d'agent oxydant, au moins un composé peroxygéné tel que défini auparavant.

Habituellement la composition est mélangée avec environ 0,5 à 10 équivalents en poids d'une composition aqueuse de peroxyde d'hydrogène.

La composition aqueuse de peroxyde d'hydrogène peut se présenter sous la forme d'une solution, une émulsion ou un gel.

En outre, la teneur en peroxyde d'hydrogène est plus particulièrement comprise entre 2 et 12% en poids de la composition aqueuse le comprenant.

Il est à noter que la composition aqueuse de peroxyde d'hydrogène présente de préférence un pH inférieur à 7 afin de garantir la stabilité de ce composé.
Cette valeur de pH est obtenue de manière classique au moyen d'agents acidifiants ou alcalins tels que décrits auparavant.

La composition aqueuse de peroxyde d'hydrogène peut encore contenir des agents conservateurs, des colorants, des parfums, des agents antimousse, des agents stabilisants du peroxyde d'hydrogène tels que notamment

le pyrophosphate de sodium, le stannate de sodium et le salicylate de sodium ainsi que des agents séquestrants.

Comme mentionné plus haut, le mélange effectué lors de l'étape a) comprend au moins un agent alcalin. Ce dernier peut être présent dans la composition selon l'invention ou bien être ajouté lors du mélange réalisé dans l'étape a).

La teneur en agent alcalin est telle que le pH du mélange obtenu à l'issue de l'étape a) est compris entre 7 et 11,5, et plus préférentiellement entre 8 et 11.

Le temps de pause nécessaire lors de l'étape b) est en général compris entre 3 et 30 minutes, de préférence entre 5 et 20 minutes.

Par ailleurs, et à titre purement indicatif, l'étape b) est réalisée à une température comprise entre 15 et 80°C de préférence entre 20 et 40 °C.

L'étape e) du procédé est classiquement réalisée à une température comprise entre 20 et 100°C, plus particulièrement entre 20 et 80°C.

Conformément à une deuxième possibilité, le procédé selon l'invention est un procédé de déformation permanente consistant à mettre en oeuvre les étapes suivantes :

a) on applique sur des fibres kératiniques préalablement mises en forme avec une composition comprenant au moins un agent réducteur et éventuellement rincées, une composition selon l'invention pendant une durée suffisante pour avoir l'effet de fixation de la forme ;
b) on rince éventuellement les fibres,
c) éventuellement on lave les fibres et on les rince,
d) on sèche ou on laisse sécher les fibres.

Tout d'abord, la première étape est réalisée sur des fibres ayant au préalable été traitées avec une composition réductrice comprenant au moins un agent réducteur.

Habituellement, l'agent réducteur est choisi parmi les réductones, les réducteurs soufrés, comme notamment les composés comprenant au moins une fonction thiol, (di)sulfure, (di)sulfite ou hydrosulfite.

Comme exemples de composés soufrés convenables, on peut citer l'acide thioglycolique, l'acide thiolactique, leurs sels de métal alcalin ou alcalino-terreux (comme le sodium, le potassium, le calcium) et leurs esters ; β-mercaptoéthanol; la cystéine, la cystéamine et leurs dérivés ; l'homocystéine et l'un de ses sels ; le mercaptoaldéhyde ; la pénicillamine ; le glutathione, la cystine ; les sulfite, bisulfite, hydrosulfite et métabisulfite de métal alcalin, alcalino-terreux ou d'ammonium ; leurs mélanges.
Parmi les réductones on peut mentionner l'acide ascorbique, l'acide isoascorbique, leurs sels ou esters ; l'hydroxypropanedial, le 2,3-hdyroxy-2-cyclopentène-1-one.

En général, la teneur en agent réducteur dans la composition réductrice représente de 1 à 30 % en poids, de préférence de 5 à 20 % en poids, par rapport au poids de la composition réductrice.

La composition réductrice peut comprendre de l'eau ou un mélange d'eau et d'un ou plusieurs solvants. Ce qui a été indiqué auparavant concernant la liste de solvants présents dans les compositions oxydantes selon l'invention comprenant de l'eau reste valable et ne sera pas repris à nouveau.

Pendant l'étape de réduction, les fibres kératiniques peuvent être mises en forme, en d'autres termes lissées ou mises sous tension au moyens de bigoudis, avant, après l'application de la composition réductrice, ou bien encore simultanément.

Le temps de pause est en général compris entre 3 et 30 minutes, de préférence entre 5 et 20 minutes.

Par ailleurs, et à titre purement indicatif, l'étape a) est habituellement réalisée à une température comprise entre 15 et 60°C, de préférence entre 20 et 40 °C.

Une fois l'étape de réduction effectuée, on rince de préférence les fibres à l'eau, puis on applique la composition oxydante selon l'invention.

La composition oxydante mise en oeuvre lors de cette étape est avantageusement une composition aqueuse. Ainsi, au cas où la composition oxydante mise en oeuvre se trouve sous une forme dépourvue d'eau ou qui présente une faible teneur en eau, ladite composition est mélangée avant l'application sur les fibres à une solution aqueuse. Cette solution aqueuse peut ou non comprendre au moins un agent alcalin tel que ceux cités auparavant.

Le temps de pause nécessaire lors de l'étape a) de fixation de la forme est en général compris entre 3 et 30 minutes, de préférence entre 5 et 20 minutes.

Par ailleurs, et à titre purement indicatif, l'étape a) est habituellement réalisée à une température comprise entre 15 et 80°C, de préférence entre 20 et 40 °C.

Conformément à une troisième variante, le procédé selon l'invention est un procédé de coloration consistant à mettre en oeuvre les étapes suivantes :

a) on mélange avant l'application sur les fibres kératiniques, une composition tinctoriale comprenant au moins un

précurseur de colorant d'oxydation et/ou au moins un colorant direct, avec une composition aqueuse selon l'invention, en présence d'un agent alcalin ;
b) on applique sur les fibres kératiniques, sèches ou humides, le mélange obtenu à l'étape précédente, et on laisse pauser pendant une durée suffisante pour avoir l'effet de coloration souhaité,
c) on rince éventuellement les fibres,
d) éventuellement on lave les fibres et on les rince,
e) on sèche ou on laisse sécher les fibres.

Lors de l'étape a), la composition oxydante selon l'invention comprend plus particulièrement comprenant en tant qu'agent oxydant, du peroxyde d'hydrogène, avec au moins un polymère conducteur, telle que décrite précédemment.
Cette composition oxydante est mélangée à une composition tinctoriale comprenant au moins un précurseur de colorant d'oxydation et/ou un colorant direct.
Les colorants précurseurs d'oxydation entrant dans la composition tinctoriale destinée à être mélangée à la composition selon l'invention, sont des composés classiquement utilisés dans le domaine et comprennent au moins un base d'oxydation éventuellement associée à au moins un coupleur.
Parmi les bases d'oxydation, on peut citer les ortho- et para-phénylènediamines, les bases doubles, les ortho- et para- aminophénols, les bases hétérocycliques suivantes ainsi que leurs sels d'addition avec un acide.
De manière classique, et si ces composés sont présents, la teneur en base d'oxydation représente de 0,0005 à 12 % en poids, de préférence de 0,005 à 8 % en poids, par rapport au poids de la composition tinctoriale.
Pour ce qui concerne plus particulièrement les coupleurs, on peut notamment citer les méta-aminophénols, les méta-phénylènediamines, les métadiphénols, les naphtols et les coupleurs hétérocycliques et leurs sels d'addition avec un acide.
Lorsqu'ils sont utilisés, la teneur en coupleur varie souvent de 0,0001 à 10 % en poids, , de préférence de 0,005 à 5 % en poids, par rapport au poids de la composition tinctoriale.
En ce qui concerne les colorants directs, ces derniers peuvent être choisis parmi les colorants directs non ioniques, cationiques ou anioniques.
Généralement, ces colorants directs sont choisis parmi les colorants benzéniques nitrés, les colorants azoïques, azométhiniques, méthiniques, anthraquinoniques, naphtoquinoniques, benzoquinoniques, phénotiaziniques indigoïdes, xanthéniques, phénanthridiniques, phtalocyanines, ceux dérivés du triarylméthane, ainsiq ue des colorants naturels, seuls ou en mélanges.
De plus, si la composition tinctoriale comprend de tels composés, leur teneur représente de 0,0005 à 12 % en poids, de préférence de 0,005 à 6 % en poids, par rapport au poids de la composition tinctoriale.
La composition tinctoriale comprend un milieu cosmétiquement acceptable qui de manière avantageuse comprend de l'eau ou un mélange eau et solvant.
La composition tinctoriale de plus des additifs classiques, par ailleurs antérieurement connus dans le traitement des fibres kératiniques humaines, tels des agents tensioactifs bien connus de l'état de la technique et de type anionique, cationique, non ionique, amphotère, zwittérionique ou leurs mélanges, des agents épaississants, des agents antioxydants, des parfums, des agents dispersants, des agents de conditionnement dont notamment des polymères cationiques ou amphotères, des agents opacifiants, des agents séquestrants tel que l'EDTA et l'acide étidronique, des filtres UV, des cires, des silicones volatiles ou non, cycliques ou linéaires ou ramifiées, organomodifiées (notamment par des groupements amines) ou non, des agents conservateurs, des céramides, des pseudocéramides, des huiles végétales, minérales ou de synthèse, les vitamines ou provitamines comme le panthénol, des polymères associatifs non-ioniques, anioniques, amphotères ou cationiques.
Comme mentionné plus haut, le mélange de la composition tinctoriale et de la composition selon l'invention a lieu en présence d'un agent alcalin.
Ce qui a été indiqué dans le cadre des autres variantes de procédé selon l'invention, de même que les conditions de pH restent valables et l'on pourra s'y référer.
Toutefois, de manière avantageuse il est à noter que l'agent alcalin est présent dans la composition tinctoriale.
La température à laquelle la composition est appliquée lors de l'étape b) est généralement comprise entre 15 et 80°C et plus particulièrement entre 15 et 40°C.
Le temps de pause est quant à lui, habituellement compris entre 5 et 60 minutes environ et plus particulièrement entre 5 et 40 minutes.
Une fois cette étape réalisée, les fibres peuvent éventuellement être rincées, puis lavées, de préférence avec un shampooing, puis rincées. Enfin, on sèche les fibres ou on les laisse sécher.
Les exemples suivants illustrent l'invention sans en limiter la portée.

**EXEMPLE**

**Synthèse de poly(thiophène-3-acide acétique)**

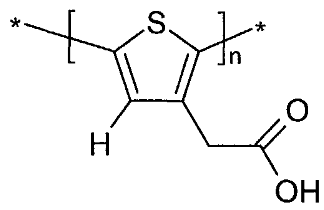

Mode opératoire

Préparation du polymère : poly(thiophène-3-acétate d'éthyle)

Dans un schlenk sous argon, on introduit 25 ml de chloroforme sec, on dégaze puis on introduit les réactifs : 2,5g de thiophène-3-acétate d'éthyle (14,7 mmol)

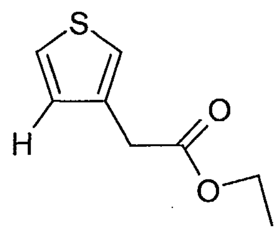

et 1 g de FeCl3 (6,15 mmol).

Le mélange est agité pendant 24 heures sous argon à 50°C.
Le polymère poly(thiophène-3-acétate d'éthyle) est alors précipité dans l'heptane.
Le polymère est ensuite dissous dans une solution de tétrahydrofuranne

Caractérisation par infra rouge :

Bande du C=O : 1719 cm-1 ; bandes du CH2, CH3 = 2979 $cm^{-1}$, 2934 cm-1 et disparition de la bande CH à 3102 $cm^{-1}$ présente dans le monomère.

**Hydrolyse du polymère:** poly(thiophène-3-acétate d'éthyle) pour former le poly(thiophène-3-acide acétique). Le polymère obtenu précédemment est alors hydrolysé par un excès de 50 ml d'une solution aqueuse d'hydroxyde de sodium (2N) pendant 48heures à 70°C, suivi d'une acidification par HCl concentré jusqu'à précipitation du produit : poly(thiophène-3- acide acétique).

Le polymère est ensuite filtré et lavé à plusieurs reprises par de l'eau distillée afin d'éliminer les traces de catalyseur.

Caractérisation du polymère Infra-rouge :

Bande du C=O : 1740 $cm^{-1}$; COO 1580 cm-1 ; OH (bande large 3000-3500 $cm^{-1}$)

Neutralisation du polymère poly(thiophène-3- acide acétique) :

Le polymère poly(thiophène-3- acide acétique) (2g) est dissous dans le tétrahydrofuranne (30g) et neutralisé à raison de 1 mol d'hydroxyde de sodium par mole d'acide carboxylique.
L'eau (30 g) est ensuite additionnée.
Le tétrahydrofuranne est évaporé.

Il est ainsi obtenu une solution aqueuse 6% de poly(thiophène-3- acide acétique) sous forme d'un sel de sodium.

| **Formulation comprenant le polymère et procédé la mettant en oeuvre.** | |
|---|---|
| Poly(thiophène-3-acide acétique) | 5 g |
| Eau oxygénée qs | 20 volumes |
| Ammoniaque à 20% qs | 9 g |
| Alcool éthylique | 20 g |
| Eau qs | 100 g |

La formule est déposée sur cheveux foncés. Après 20 minutes d'attente, on procède au séchage (séchage libre).

## Revendications

**1.** Composition comprenant, dans un milieu cosmétiquement acceptable,

(a) au moins un agent oxydant,
(b) au moins un polymère conducteur.

**2.** Composition selon la revendication précédente, **caractérisé en ce que** le polymère conducteur comprend au moins une unité répétitive de formules suivantes :

<u>les anilines de structure (I) suivante</u> :

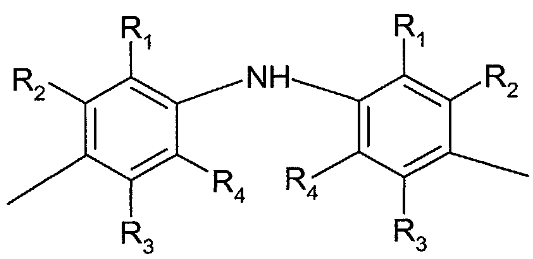

(I)

<u>les pyrroles de structure (IIa) et (IIb) suivantes :</u>

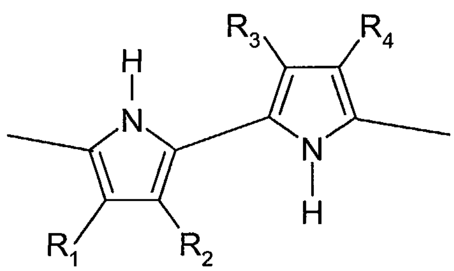

(IIa)

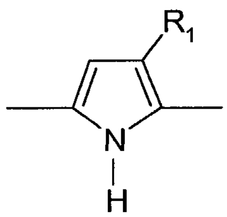

(IIb)

les thiophènes ou bisthiophènes de formules (IIIa), (IIIb) et (IIIc) suivantes :

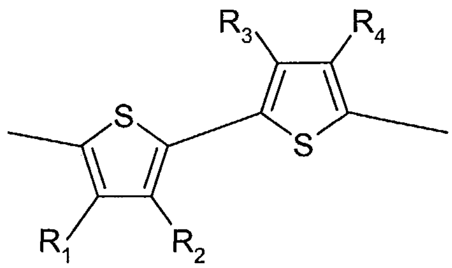

(IIIa)

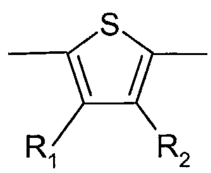

(IIIb)

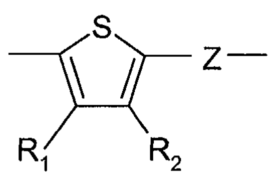

(IIIc)

les furanes de formule (IV) suivante :

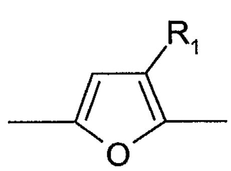

(IV)

les para-phénylène-sulfure de structure (V) suivante :

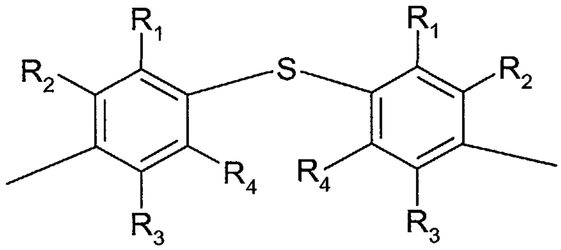

(V)

les para-phénylène vinylène de formule (VI) suivante :

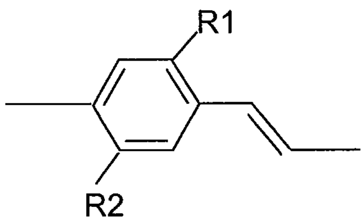

(VI)

les indoles de formule (VII) suivante :

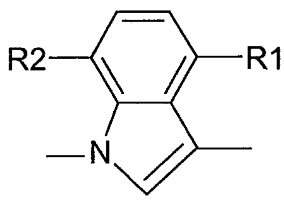

(VII)

les amides aromatiques de formules suivantes (VIIIa), (VIIIb), (VIIIc), (VIIId) :

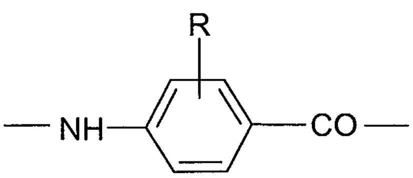

(VIIIa)

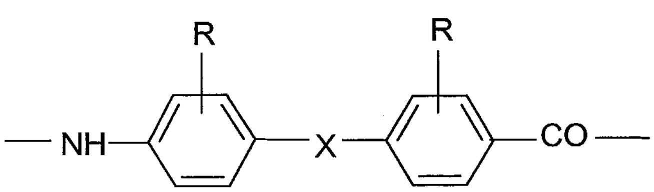

(VIIIb)

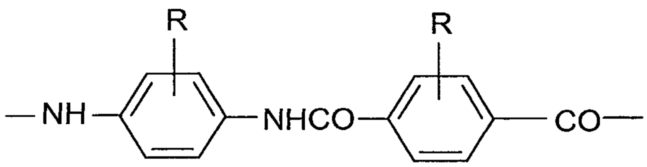

(VIIIc)

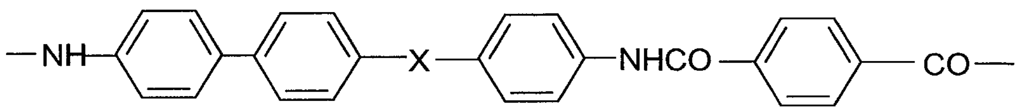

(VIIId)

les hydrazides aromatiques de formules (IXa), (IXb) et (IXc) suivantes :

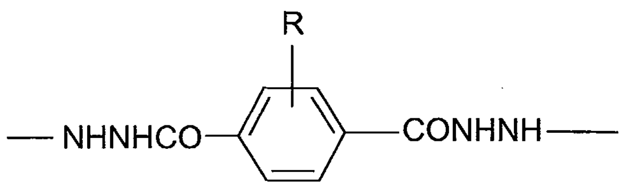

(IXa)

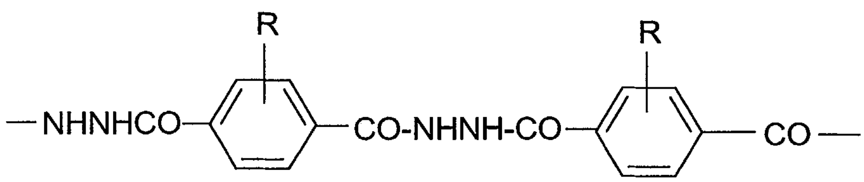

(IXb)

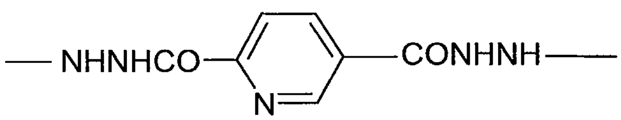

(IXc)

les azométhines aromatiques de formules (Xa), (Xb) et (Xc) suivantes :

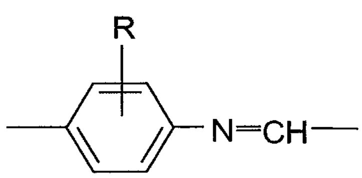

(Xa)

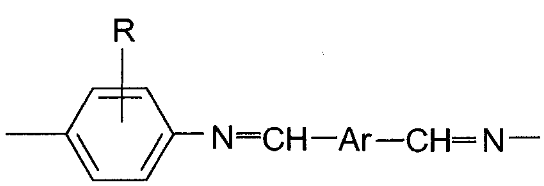

(Xb)

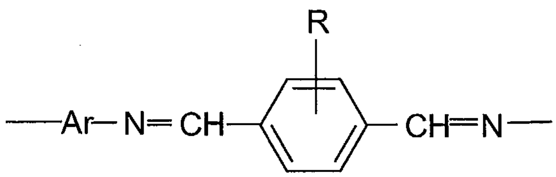

(Xc)

<u>les esters aromatiques de formules (XIa), (XIb) et (XIc) suivantes :</u>

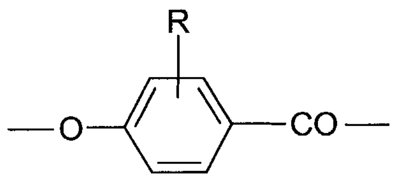

(XIa)

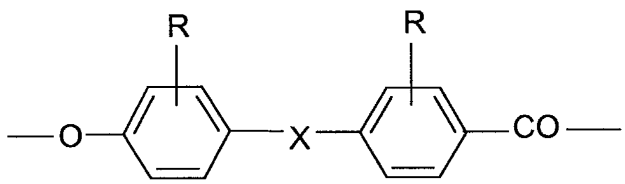

(XIb)

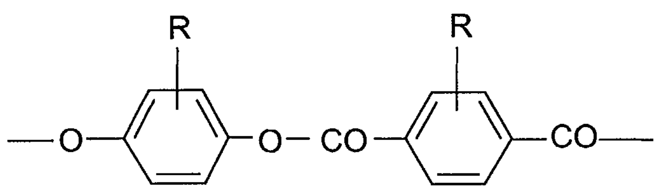

(XIc)

formules (I) à (XI) dans lesquelles :

les radicaux R, $R_1$ à $R_4$ identiques ou différents, sont choisis dans le groupe formé par l'hydrogène, un radical -R', -OR', -COOR', -OCOR', avec R' représentant un radical alkyle linéaire ou ramifié en $C_1$-$C_{20}$, un atome d'halogène, un radical nitro, un radical cyano, un radical cyanoalkyle, et des groupements solubilisants;
Ar représente un radical comprenant un radical monoaromatique ou polyaromatique X = -NHCO-, -O-, -S-, $-SO_2-$, -N=N-, $-C(CH_3)_2-$, $-CH_2-$, -CH=CH-, -CH=N- ;
Z = —CH=CH— ou —C≡C—.

**3.** Composition selon l'une des revendications 1 ou 2, **caractérisée en ce que** les groupements solubilisants sont choisis dans le groupe formé par :

- un radical carboxylique (-COOH), carboxylate ($-COO^-M^+$ avec M représentant un métal alcalin, un métal alcalino-terreux, une amine organique, une alcanolamine, un acide aminé),
- un radical sulfonique ($-SO_3H$), sulfonate ($-SO_3^-$ $M^+$, M ayant la même définition que ci-dessus),
- un radical amine primaire, secondaire, tertiaire,
- un radical ammonium quaternaire tel $-NR'_3{}^+$ $Z^-$ avec Z= Br, Cl, alkyl($C_1$-$C_4$)-$OSO_3$ et R' alkyles identiques ou non, linéaires ou ramifiés en $C_1$ à $C_{20}$, ou formant un hétérocycle avec l'azote pour deux d'entre eux,
- un radical hydroxyle,
- un radical polyoxyde d'alkylène en $C_2$-$C_3$.

**4.** Composition selon l'une quelconque des revendications précédentes, **caractérisée en ce que** les groupements solubilisants sont reliés au cycle par l'intermédiaire d'un groupement espaceur tel que par exemple un radical -R"-,

-OR"-, - OCOR"- ou -COOR"- avec R" représentant un radical alkyle, linéaire ou ramifié en $C_1$-$C_{20}$, comprenant éventuellement un ou plusieurs hétéroatomes.

5. Composition selon l'une quelconque des revendications précédentes, **caractérisée en ce que** les radicaux R, $R_1$ à $R_4$ identiques ou différents, sont choisis parmi l'hydrogène, R', -OR', -OCOR', -COOR' avec R' représentant un radical alkyle linéaire ou ramifié en $C_1$-$C_6$, et parmi les groupements solubilisants suivants, neutralisés ou non : -COOH, $-CH_2COOH$, $-CH_2OH$, $-(CH_2)_6OH$, $-(CH_2)_3SO_3H$, $-O(CH_2)_3SO_3H$, - $O(CH_2)_3N(CH_2CH_3)_2$, $-[(CH_2)_2O]xCH_2CH_2OH$, $-[(CH_2)_2O]xCH_2CH_2OCH_3$ avec x nombre moyen compris entre 0 et 200.

6. Composition selon l'une quelconque des revendications précédentes, **caractérisée en ce que** le polymère est tel qu'au moins un radical parmi R, R1 à R4 désigne un groupement solubilisant.

7. Composition selon l'une quelconque des revendications précédentes, **caractérisée en ce que** le polymère conducteur comporte au moins un groupement solubilisant par unité répétitive.

8. Composition selon l'une quelconque des revendications précédentes, **caractérisée en ce que** les groupements solubilisants sont choisis parmi les groupements acide carboxylique ; acide sulfonique ; les radicaux amine tertiaire; ammonium quaternaire tels que $-NR'_3{}^+ Z^-$ avec Z= Br, Cl, alkyl($C_1$-$C_4$)-$OSO_3$ et R', identiques ou non, représentant un radical alkyle linéaire ou ramifié en $C_1$-$C_{20}$ ; éventuellement relié au cycle par un espaceur, de préférence un radical alkyle en $C_1$-$C_{20}$ ; ainsi que leurs sels ; les fonctions acides carboxyliques ou sulfoniques peuvent ou non être neutralisées.

9. Composition selon l'une des revendications précédentes, **caractérisé en ce que** le polymère conducteur correspond aux formules (IIIa), (IIIb) ou (IIIc), dans lesquelles au moins un radical R1 à R4 de la formule (IIIa) ou R1 ou R2 des formules (IIIb) ou (IIIc) représente un groupement solubilisant du type acide carboxylique, sous forme neutralisée ou non, éventuellement relié au cycle par un espaceur, de préférence un radical alkyle linéaire ou ramifié en C1-C20, le ou les autres radicaux représentant un atome d'hydrogène.

10. Composition selon l'une quelconque des revendications précédentes, **caractérisée en ce que** le ou les polymères conducteurs sont présents dans des proportions d'au moins 0,001 % en poids par rapport au poids total de la composition.

11. Composition selon l'une quelconque des revendications précédentes, **caractérisée en ce que** le ou les polymères conducteurs sont présents dans des proportions d'au plus 50 % en poids par rapport au poids total de la composition.

12. Composition selon l'une quelconque des revendications précédentes, **caractérisée en ce que** le teneur en polymère conducteur représente 0,1 à 50 % en poids par rapport au poids total de la composition.

13. Composition selon l'une quelconque des revendications précédentes, **caractérisée en ce que** l'agent oxydant est choisi parmi les composés peroxygénés ; les dérivés halogénés; les ferricyanures de métaux alcalins ; les enzymes, seuls ou en mélanges.

14. Composition selon l'une quelconque des revendications précédentes, **caractérisée en ce que** l'agent oxydant est le peroxyde d'hydrogène.

15. Composition selon l'une des revendications 13 ou 14, **caractérisée en ce que** la teneur en agent oxydant la teneur totale en agent oxydant représente de 0,05% à 25% en poids par rapport au poids total de la composition.

16. Composition selon l'une quelconque des revendications 1 à 13 et 15, **caractérisée en ce qu'**elle comprend au moins un agent alcalin.

17. Composition selon la revendication précédente, **caractérisée en ce que** l'agent alcalin est choisi parmi l'urée, les silicates et phosphates de métaux alcalins ou alcalino-terreux, les composés précurseurs d'ammoniac.

18. Composition selon l'une quelconque des revendications 1 à 13 et 15 à 17, **caractérisée en ce qu'**elle ne comprend pas d'eau.

**19.** Composition selon l'une quelconque des revendications 1 à 13 et 15 à 17, **caractérisée en ce qu'**elle comprend de l'eau à une teneur inférieure à 1 % en poids de la composition oxydante.

**20.** Composition selon l'une quelconque des revendications 1 à 17, **caractérisée en ce que** la composition comprend un milieu cosmétiquement acceptable qui est constitué par de l'eau ou un mélange d'eau et d'un ou plusieurs solvants organiques acceptables dans le domaine.

**21.** Composition selon la revendication précédente, **caractérisée en ce que** le ou les solvants sont choisis parmi des alcools en C1-C4, des alcools aromatiques, des glycols, des éthers de glycol, des polyols, des polyéthylèneglycols, le polypropylèneglycol et leurs mélanges.

**22.** Composition selon l'une quelconque des revendications précédentes, **caractérisée en ce qu'**elle comprend en outre au moins un agent tensioactif non ionique, anionique, cationique, amphotère ou zwittérionique.

**23.** Composition selon la revendication précédente, **caractérisée en ce que** la teneur en tensioactif est de moins de 30% en poids, et de préférence comprise entre 0,5 et 10% en poids, par rapport au poids de la composition.

**24.** Composition prête à l'emploi comprenant la composition selon l'une quelconque des revendications précédente, comprenant au moins un composé peroxygéné et/ou des bromates de métaux alcalins, et du peroxyde d'hydrogène, en tant qu'agents oxydants.

**25.** Procédé de traitement des fibres kératiniques **caractérisé en ce que** l'on applique la composition selon l'une des revendications 1 à 24.

**26.** Procédé de décoloration des fibres kératiniques humaines et plus particulièrement les cheveux, **caractérisé en ce qu'**il consiste à effectuer les étapes suivantes :

a) on mélange avant l'application sur les fibres kératiniques, une composition selon l'une des revendications 1 à 13 et 15 à 23 avec une composition aqueuse comprenant du peroxyde d'hydrogène, éventuellement en présence d'un agent alcalin ;
b) on applique sur les fibres kératiniques, sèches ou humides, le mélange obtenu à l'étape précédente, et on laisse pauser pendant une durée suffisante pour avoir l'effet de décoloration souhaité,
c) on rince éventuellement les fibres,
d) éventuellement on lave les fibres et on les rince,
e) on sèche ou on laisse sécher les fibres.

**27.** Procédé de déformation permanente des fibres kératiniques humaines et plus particulièrement les cheveux, **caractérisé en ce qu'**il consiste à effectuer les étapes suivantes :

a) on applique sur des fibres kératiniques préalablement mises en forme avec une composition comprenant au moins un agent réducteur et éventuellement rincées, une composition selon l'une des revendications 1 à 24, pendant une durée suffisante pour avoir la fixation de la forme;
b) on rince éventuellement les fibres,
c) éventuellement on lave les fibres et on les rince,
d) on sèche ou on laisse sécher les fibres.

**28.** Procédé de coloration des fibres kératiniques, notamment humaines, et plus particulièrement des cheveux, consistant à mettre en oeuvre les étapes suivantes :

a) on mélange avant l'application sur les fibres kératiniques, une composition comprenant au moins un précurseur de colorant d'oxydation et/ou au moins un colorant direct, avec une composition aqueuse selon l'une des revendications 1 à 13 et 15 à 23, comprenant comme agent oxydant, du peroxyde d'hydrogène, éventuellement en présence d'un agent alcalin ;
b) on applique sur les fibres kératiniques, sèches ou humides, le mélange obtenu à l'étape précédente, et on laisse pauser pendant une durée suffisante pour avoir l'effet de coloration souhaité,
c) on rince éventuellement les fibres,
d) éventuellement on lave les fibres et on les rince,
e) on sèche ou on laisse sécher les fibres.

**29.** Utilisation de la composition selon l'une quelconque des revendications 1 à 24, pour conférer un effet optique à des fibres kératiniques.

**30.** Utilisation selon la revendication précédente, **caractérisée en ce que** l'effet optique est un effet de brillance.